# EUROPEAN PATENT SPECIFICATION

(11) **EP 2 644 171 B1**
(45) Date of publication and mention of the grant of the patent: **03.03.2021**
(21) Application number: 13275052.2
(22) Date of filing: 11.03.2013
(51) Int. Cl.: A61F 2/958, A61M 25/10

(54) **Method of manufacture of a medical balloon with textured or roughened outer layer**
Methode zu Herstellung eines medizinischen Ballons mit texturierter oder angerauter äußeren Schicht
Procédé de production d'un ballonnet médical avec couche extérieure texturée ou rugueuse

(30) Priority: 27.03.2012 GB 201205368
(43) Date of publication of application: 02.10.2013
(73) Proprietor: Cook Medical Technologies LLC, Bloomington, IN 47404 (US)
(72) Inventor: Klausen, Anders Scheel, DK-4700 Naestved (DK); Lysgaard, Thomas, 2680 Solroed Strand (DK); Torp, Allan, 4632 Bjaeverskov (DK); Sonnichsen, Anne-Mette Hoppe, 4180 Soroe (DK)
(74) Representative: Jehan, Robert

(56) References cited:
- WO-A1-00/57815
- WO-A1-00/57816
- WO-A1-2009/080320
- US-A1- 2007 142 771

## Description

### Technical Field

The present invention relates to a method of making a balloon of the type taught herein.

### Background Art

Medical balloons are used for a variety of medical procedures including angioplasty, vessel dilatation, valvuloplasty, occlusion and for many other applications. In many such applications, it is desirable to be able to position the medical balloon at a precise location and to retain it at this location during the medical procedure. However, balloons tend to have smooth exterior surfaces, which can enable them to migrate during the procedure. This can result in an ineffective medical procedure and/or potential damage to a patient's organs.

It is thus desirable to give such medical balloons a surface characteristic which assists in anchoring the balloon within the vessel wall or valve opening. One possible solution is to form a balloon with a roughened outer surface. However, prior attempts to roughen the outer surface of a balloon have generally not been successful due to the fact that inflation of the balloon tends to stretch the balloon wall and thus flatten any roughening of the outer surface.

Medical balloons are disclosed in a number of earlier publications including, for example, in US-2010/0,036,314, US-A-6,143,416, US-A-6,786,889, US-2009/0,299,450, US-5,620,649, US-7,828,766, US-2010/0,262,218, WO 2009/080320, WO 00/57816, US 2007/0142771.

### Disclosure of the Invention

The present invention seeks to provide an improved method of making a medical balloon. The invention is defined by the appended claims. There is provided the inventive method of claim 1.

Also described herein is a medical balloon provided with first and second layers, the second layer overlying the first layer, the second layer including a textured or roughened outer surface; wherein the first layer is integral with the second layer so as to support the second layer, wherein the second layer retains a textured or roughened outer surface on inflation of the balloon.

The function of the first or inner layer is to support the second or outer layer such that inflation of the balloon does not result in flattening of the texturing of the outer layer.

The first layer, being less compliant, will support and hold the inflation pressure imparted to the balloon, preventing or reducing the effect of that pressure on the outer layer.

In an embodiment, the second balloon layer has a surface texture or roughness of geometrically shaped surface features. The geometrically shaped surface features may include circumferential rings or ribs, grooves, protrusions and/or depressions, toothed elements, random protrusions.

The first and second balloon layers may be coextruded.

Advantageously, the first balloon layer has substantially smooth surfaces. This has the advantage that the inner layer will adopt its inflated form with no unfolding or flattening out of surface formations, as might occur, for example, with a layer having a corrugated shape when unpressurised.

Described herein is a medical balloon provided with first and second layers, the second layer overlying the first layer, the second layer including a textured or roughened outer surface; wherein the first layer is integral with the second layer so as to support the second layer, wherein the first balloon layer has a first compliance and the second balloon layer has a second compliance, the first compliance being less than the second compliance, wherein the first balloon layer is made of a material having a first softening or melting temperature and the second balloon layer is made of a material having a second softening or melting temperature lower than the first softening or melting temperature, wherein the
second layer retains a textured or roughened outer surface on inflation of the balloon.

Described herein is a method of forming a medical balloon provided with first and second layers, the second layer overlying the first layer, the second layer including a textured or roughened outer surface; wherein the first layer is integral with the second layer so as to support the second layer, wherein the second layer retains a roughened outer surface on inflation of the balloon; the first balloon layer being made of a material having a first softening or melting temperature and a first compliance, and the second balloon layer being made of a material having a second softening or melting temperature and a second

compliance, the second softening or melting temperature being lower than the first softening or melting temperature and the first compliance being less than the second compliance; the method including the steps of:
providing a raw tubing having first and second layers of said first and second materials respectively;
locating said raw tubing in a mold, which mold includes a textured or roughened inner surface;
inflating and heating the raw tubing so as to cause it to inflate, wherein said heating causes said second material to soften or melt;
pressing said second layer against the mold surface by means of pressure within the raw tubing, said pressing causing said second layer to acquire a textured or roughened outer surface;
said inflating, heating and pressing forming said balloon; and
cooling and deflating said balloon, wherein the second surface of the balloon retains said roughened outer surface.

### Brief Description of the Drawings

Embodiments of the present invention are described below, by way of example only, with reference to the accompanying drawings, in which:
Figure 1 is a schematic diagram of an example of balloon catheter;
Figure 2 is a cross-sectional view of an embodiment of balloon;
Figure 3 is a side elevational view of the balloon of Figure 2;
Figures 4 to 6 show cross-sectional views of various examples of surface texturing of the second layer of a medical balloon according to the teachings herein;
Figure 7 is a schematic diagram of an example of apparatus for manufacturing a balloon of a balloon catheter as taught herein;
Figure 8 is a schematic diagram of a balloon forming mold for the apparatus of Figure 7; and
Figure 9 is a schematic diagram of the preferred embodiment of raw tubing used in the manufacture of a medical balloon as taught herein.

### Description of the Preferred Embodiments

It is to be understood that the drawings are schematic only and are not intended to be representative of dimensions or proportions of the various elements shown therein. In some instances, dimensions, sizes and proportions have been modified in order to assist in the visualisation of various features of the elements shown, that is for the purpose of explanation only. The person skilled in the art will be aware of the appropriate dimensions and proportions having regard to common knowledge in the art.

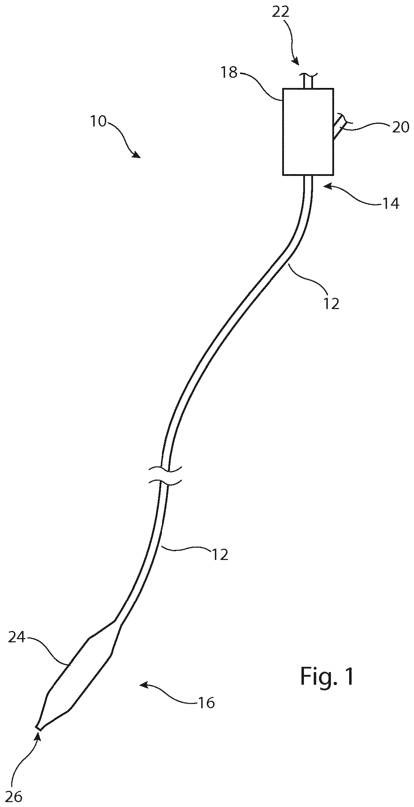

Referring to Figure 1, there is shown in schematic form the principal components of a balloon catheter, which components are generally known in the art. The balloon catheter includes a catheter 12 having a proximal end 14 and a distal end 16. At the proximal end 14, the catheter is coupled to a manipulation unit and valve assembly 18, which typically includes one or more haemostatic valves (not shown), a port 20 for feeding flushing liquid into the catheter 12, typically saline solution, and a proximal cannula 22 for use, for example, in feeding a guide wire (not shown) through the catheter 12.

At the distal end 16 of the balloon catheter 20, there is provided a medical balloon 24. The balloon 24 may be used, for example, in an angioplasty or other vessel dilatation procedure, for valvuloplasty, for occlusion or for any other procedure. The balloon 24 is typically wrappable around the catheter 12, the latter extending through the balloon 24 to the tip 26 of the assembly 10. The balloon is also inflatable, via an inflation lumen in the catheter 12, so as to attain a deployed, inflated configuration, as shown in Figure 1. The balloon 24 may have a variety of shapes but typically may have a substantially cylindrical body portion bounded by conical end portions which themselves are bounded to neck portions which are fixed in fluid tight manner to the catheter 12.

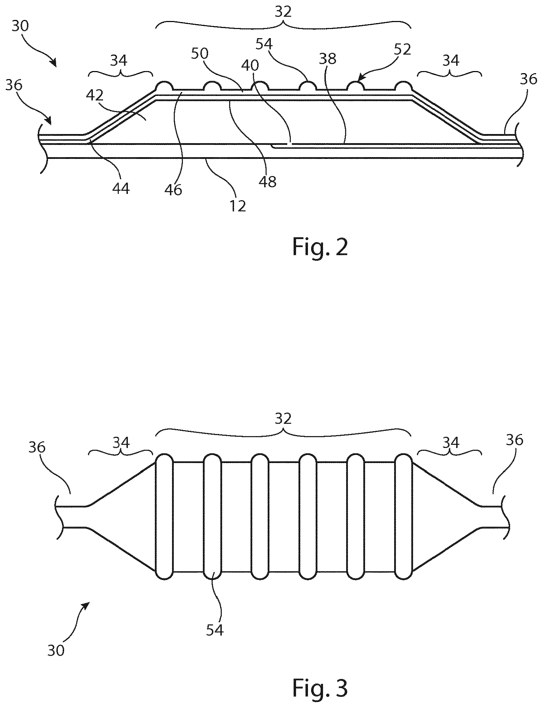

Referring now to Figures 2 and 3, there is shown a first embodiment of medical balloon according to the teachings herein. Figure 2 is cross-sectional view of a part of the balloon 30 taken along the longitudinal axis of the balloon. Figure 3, on the other hand, is a view of the balloon 30 along its longitudinal axis. The balloon 30 has a body portion 32 which is substantially cylindrical. This is bounded by first and second conical ends 34 which in turn terminate at first and second necks 36 are typically bonded or otherwise sealed to the catheter 12. It will be apparent, in particular from Figure 2, that catheter 12 extends through the balloon 30 and is provided, typically, with an inflation lumen 38 which has a port 40 in communication with the internal chamber 42 of the balloon 30. Inflation lumen 38 is used to inflate and deflate the balloon 30 for deployment purposes, in a manner well known in the art.

As can be seen in Figure 2, the balloon 30 is formed, in this example, of first and second walls or layers 44, 46. The inner layer 44 has a substantially smooth inner surface 48 and, preferably, a substantially smooth outer surface 50. Typically, the inner layer 44 has a substantially even thickness, although it is not excluded that it may have a non-uniform thickness. Similarly, the end cones 34 and/or the necks 36 may have a thickness which varies, for example as a result of the method of manufacture of the balloon.

Integral with or otherwise bonded to the inner layer 44 is the second or outer layer 46. This is typically in continuous and complete contact with the inner layer 44 and thus has an inner surface which is consistent in shape and size with the outer surface 50 of the inner layer 44. In this example, the inner surface 50 of the outer layer 46 is smooth as is the outer surface of the inner layer 44. On the other hand, the outer surface 52 of the outer layer 46 is textured. In this example, the outer surface 52 of the outer layer 46 is provided with a plurality of circumferential ribs or rings 54 which extend substantially transversally to the longitudinal axis of the balloon 30. The ribs or rings 54 can clearly be seen in Figure 3. In this example, the ribs 54 are located on the body portion 32 of the balloon 30 but in other embodiments may extend over only part of the body portion and equally in some embodiments may extend along at least the end cones 34 and possibly also along the necks 36. The nature, location, size and positioning of the ribs 54 can be determined by a person skilled in the art on the basis of the particular medical application.

The texturing could take other forms, including for example randomly oriented ribs or elongate protrusions disposed over the outer surface of the balloon in what could be termed a mottled arrangement. Similarly, the texturing could be in the form of a lattice network of ribbing, arranged parallel and perpendicular to the longitudinal dimension or axis of the balloon, arranged at angles, for example 45 degrees, thereto or at other angles. Similarly, in one embodiment, the texturing could be in the form of wave-shaped ribs extending along the balloon, longitudinally, circumferentially or at another angle. Other designs of ribbing could be used as well.

It is preferred that the texturing or roughening is between 0.2 Ra to 18 Ra at an inflation pressure from 1 atm (101.325 kPa) to 25 atm (2533 kPa). It is to be understood that the limits of these ranges may be extended within normal parameters without loss of functionality.

In an embodiment, the inner layer of the balloon has a thickness of around 0.01 millimetres to around 0.10 millimetres, while the outer layer has a thickness or around 0.005 millimetres to around 0.05 millimetres.

The inner layer 44 of the balloon 30 can be made of a variety of materials including, for example, polyamide (e.g. Nylon), polyether block amide (e.g. Pebax), polyethylene, PET, polyurethane or other suitable material. The second or outer balloon layer 46 could be made of similar materials or a different material than that of the inner layer 44, all being of a formulation having a lower softening or melting temperature than the material of the first balloon layer. Of course, either or both layers 44, 46 can be made from a plurality of compounds.

In the preferred embodiment, the balloon layers have softening temperatures between around 60 and 160 degrees centigrade with a difference in the softening temperatures of the inner and outer layers of a few tens of degrees centigrade, in one embodiment of around 60 degrees centigrade.

The inner layer 44 is made of a material having a higher softening or melting temperature than the material forming the outer layer 46. It is also preferred that the outer layer 46 has a compliance which is no less than the compliance of the inner layer 44 and most preferably has a greater compliance than that of the inner layer 44. In particular, it is preferred that the outer layer has a softer/lower durometer that the inner layer.

The structure of layers 44, 46 is such that when the balloon 30 is inflated, by means of inflation fluid fed through the lumen 38 of the catheter 12, the balloon 30 will unwrap from the catheter 12 and expand to its inflated condition shown in Figures 2 and 3. The inner layer 44 will take the inflation pressure of the inflation fluid and as a result of having a compliance which is the same as or less than the compliance of the outer layer 46, will take the bulk of the stress caused by that inflation pressure. In other words, any stretching of the balloon 30 as a result of the inflation pressure will be predominantly taken by the inner layer 44, with the result that the inner layer 44 ensures that the outer layer 46 undergoes reduced or otherwise minimised amounts of stretching. As a result, the outer layer 46 will be stretched and thus flattened much less than can occur with prior art balloon structures. In other words, any surface texturing of the outer surface 52 of the outer layer 46 will tend to be maintained.

The relative compliances of the inner and outer layers 44, 46 can be determined by a number of factors, including the material used for these layers, the nature of that material, the thicknesses of the layers and so on. These are all parameters which a person skilled in the art will be able to ascertain on the basis of common general knowledge.

The ribs 54 of the embodiment of Figures 2 and 3 provide a texturing of the outer surface of the balloon which can assist in holding the balloon 30 in position within a patient's vessel, valve or other organ, thereby to reduce or prevent likelihood of the balloon 30 migrating or otherwise shifting during a medical procedure.

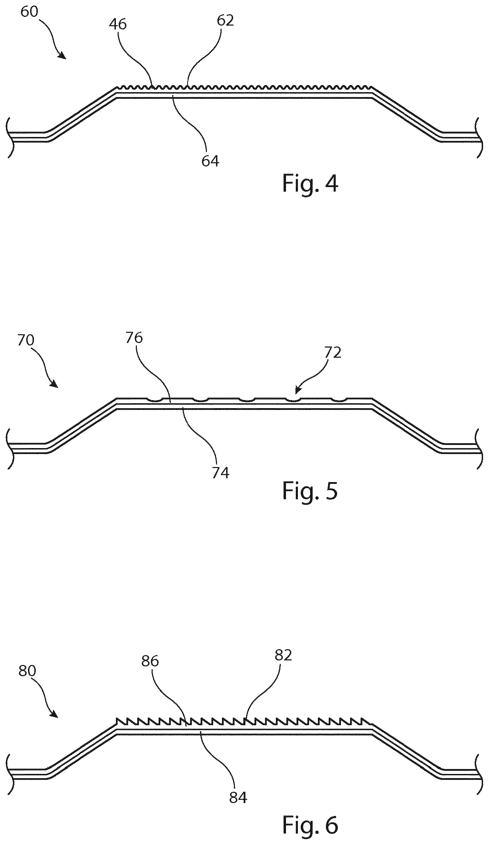

It is to be appreciated that the circumferential ribs or rings 54 are just one example of texturing of a medical balloon. The teachings herein, particularly in connection with the inventive method of manufacture of the balloon described below, allow for a large variety of different surface features to be produced in a medical balloon. Some examples are given in Figures 4, 5 and 6, to which reference is now made. All of these Figures show cross-sectional views of different examples of medical balloon.

In Figure 4, the balloon 60 is roughened, by means of creation of a roughened outer surface 62 and the outer layer 46 of the balloon 30. As with the embodiment of Figures 2 and 3, the balloon 60 includes an inner layer 64 having the same characteristics of the inner layer 44 of the example of Figures 2 and 3. The roughening 62 could be random, in the form of pits and protrusions of random sizes and/or shapes and/or positions along the body portion of the balloon 60.

In Figure 5, the balloon 70 shown is provided with a series of circumferential grooves 72, formed in the outer surface of the outer layer 76. As with the embodiments, the balloon 70 includes an inner layer 74 of the type disclosed herein.

Figure 6 shows another example of balloon 80 having a toothed surface formed of the outer surface 82 of the outer layer 86 of the balloon 80. The teeth 82 could be pointed in a distal direction or in a proximal direction and in some embodiments there could be teeth pointing in both directions, for instance towards their closest balloon end. The balloon 80 has an inner layer 84 of the characteristics taught herein.

These texturing features could be used individually or in combination with one another and may also extend down the end cones of the balloon, and in some embodiments also along the neck portions.

The balloon could be non-compliant, semi-compliant or compliant in dependence upon the medical application.

The structure of the balloon and its method of manufacture, described below, allows for the provision of medical balloons having a large variety of surface textures or roughnesses, the specific characteristics of which can be designed to be suited or otherwise optimised for a particular medical application.

There follows a description of a preferred embodiment of manufacturing a balloon having characteristics of the type disclosed herein.

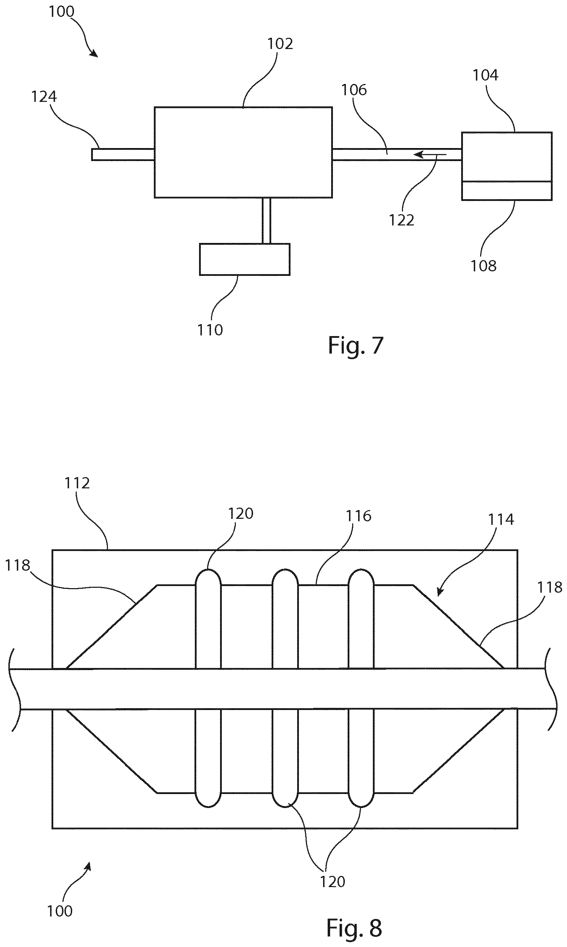

Referring now to Figure 7, there is shown in schematic form an example of assembly 100 for use in the manufacture of medical balloon and balloon catheters of the types disclosed herein.

The assembly 100 includes a mold 102, a pumping unit 104 for pumping inflation fluid through a conduit 106 into the mold 102 and specifically into a raw tubing from which the medical balloon is formed as described in further detail below. The pumping unit 104 may be provided with a heater 108 for heating the pumping fluid. There may be provided a separate heating unit 110 for heating the mold 102 during the process of fabrication of a medical balloon.

With reference to Figure 8, there is shown a cross-sectional view of an example of mold 112 shaped to produce a medical balloon having a series of circumferential ribs or rings on the outer surface of the balloon. For this purpose, the mold 112 has an internal wall 114 with a substantially cylindrical surface 116 bounded by tapering sections 118 which in practice will form the end cones 34 of the balloon.

Formed within the substantially cylindrical portion 116 of the mold are annular grooves 120 which extend transversally around the inside surface of the cylindrical portion 116.

In the example of Figure 8, the mold 100 is longitudinally divided in at least two portions forming half a mold each. The format of the mold 100 is not, however, relevant to the disclosure herein in that the mold 100 could have sections divided in other ways, for example transversally rather than longitudinally, in order to gain access to the inside of the mold for the purposes of removing a balloon formed therewithin.

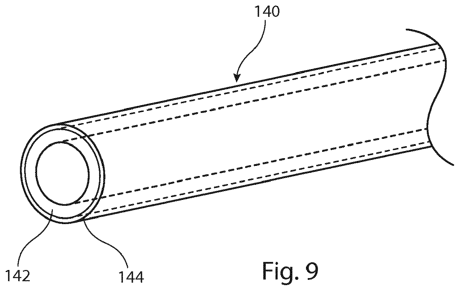

Referring now to Figure 9, there is shown an example of raw tubing 140 used in making a medical balloon of any of the types disclosed herein. The raw tubing 140 is formed, for example by co-extrusion, of two layers 142 and 144. The inner layer 142 forms the inner layer 44, 64, 74, 84 of the balloon, whereas the outer layer 144 forms the outer layer 46, 66, 76 or 86 of the balloon. These layers are thus made of the same material as the eventual layers of the balloon.

In practice, the raw tubing 140 is fed into the mold 102, typically in the direction of the arrow 122 shown in Figure 7 so as to extend into and through the mold 102. Once so fed, the raw tubing 140 is suitably clamped into the mold and closed off at its extreme end indicated by reference numeral 124 in Figure 7. The fixing is such as to seal the end 124 in fluid tight manner. This arrangement is known in the art and will thus be immediately evident to the skilled person.

The tubing 140, which is typically a very long or continuous length of tubing, is cut to an appropriate length and then coupled to the conduit 106, in known manner. In practice, the conduit 106 may form a balloon catheter 12, in which case the raw tubing 140 would be fixed over the catheter after having been cut to size with its two ends sealed to the catheter 12 at locations which would form the necks 36 of the balloon.

The mold 102 is heated and fluid pressure, typically also heated, fed by means of the pump 104 into the raw tubing 140. The heat applied to the raw tubing causes this to soften, while the pressure of the inflation fluid causes the raw tubing to expand within the chamber of the mold 102. As the raw tubing 140 expands towards the internal wall 114 of the mold 112, the raw tubing 140 will eventually be pressed against these walls by continuing inflation pressure. The outer layer 144 of the raw tubing, being of a softer material, will take the shape of any texturing or roughening on the internal walls 114 of the raw tubing, in this case of the circumferential grooves 120 within the cylindrical portion 116 of the mold 112. On the other hand, the inner layer 142, being preferably of a less conformable material, will remain substantially flat, that is will not deform to take any of the shape of the texturing or roughening on the internal walls of the mold 112. As a result, the raw tubing 140 takes shapes similar to those shown in Figures 2 and 4 to 6 of the accompanying drawings.

Once fully inflated, the mold 112 is cooled or allow to cool and the balloon then removed from the mold. Typically, this can be achieved by deflating the balloon so as to facilitate its retraction form the mold surfaces.

The provision of two layers to the balloon integral with one another enables the inner layer to be substantially flat (that is not having any texturing or roughening) and the second layer to exhibit surface texturing or roughening. The smooth inner layer will then act to support the outer layer upon an inflation of the balloon and to reduce or prevent flattening of the texturing or roughening on the outer surface of the second layer. This can be particularly ensured by use of an outer layer which has a lower melting or softening point on the inner layer and thus in which the inner layer will act to provide support to the outer layer, both during the manufacture of the balloon and also during subsequent deployment of the balloon in a medical procedure.

The preferred embodiments have only two balloon layers, which are preferably co-extruded or otherwise bonded to one another so as to be integral. Other embodiments contemplate more than two layers, for example, three or more, with the proviso that the outer layer of the balloon remains supported by an internal layer which prevents or minimises stretching of the outer layer which would lead to flattening of any texturing or roughening on its outer surface.

The roughened or textured outer surface of the balloon can also be used advantageously for holding an implantable medical device securely on the balloon for delivery of the device. More particularly, the roughening or texturing will act to minimise or prevent slippage of a medical device supported on the balloon while this is being deployed. Smooth balloons can sometimes allow the medical device to slip thereon, resulting in incorrect deployment of the device. For this purpose, the outer layer of the balloon may be relatively soft to allow for partial embedding of the medical device into the layer, which will enhance the grip on the device. This feature can be particularly advantageous for the deployment of balloon expandable stents, stent grafts, and so on.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. A method of forming a medical balloon (30, 60, 70, 80) provided with first (44, 64, 74, 84) and second layers (46, 66, 76, 86), the second layer overlying the first layer, the second layer including a textured or roughened outer surface; wherein the first layer is integral with the second layer so as to support the second layer, wherein the second layer retains a roughened outer surface on inflation of the balloon; the first balloon layer being made of a material having a first softening or melting temperature and a first compliance, and the second balloon layer being made of a material having a second softening or melting temperature and a second compliance, the method including the steps of:
providing a raw tubing (140) having first (142) and second layers (144) of said first and second materials respectively;
locating said raw tubing in a mold (102), which mold includes a textured or roughened inner surface;
inflating and heating the raw tubing so as to cause it to inflate, wherein said heating causes said second material to soften or melt;
pressing said second layer against the mold surface by means of pressure within the raw tubing, said pressing causing said second layer to acquire a textured or roughened outer surface;
said inflating, heating and pressing forming said balloon; and
cooling and deflating said balloon, wherein the second surface of the balloon retains said roughened outer surface,
said first balloon layer and said second balloon layer **characterized in that** the second softening or melting temperature is lower than he first softening or melting temperature and **in that**
the first compliance is less than the second compliance.

2. A method according to claim 1, wherein the second balloon layer has a surface texture or roughness of geometrically shaped surface features.

3. A method according to claim 2, wherein the geometrically shaped surface features include circumferential rings or ribs, grooves, protrusions and/or depressions, toothed elements, random protrusions.

4. A method according to any preceding claim, wherein the roughness or texturing of the outer surface of the balloon is between 0.2 Ra to 18 Ra at an inflation pressure form 1 atm to 25 atm.

5. A method according to any preceding claim, wherein the first and second balloon layers are coextruded.

6. A method according to any preceding claim, wherein the first balloon layer has substantially smooth surfaces.

7. A method according to any preceding claim, wherein the second balloon layer has a substantially smooth inner surface.

8. A method according to any preceding claim, wherein the balloon is formed of two balloon layers.

9. A method according to any preceding claim, wherein the first and second balloon layers are directly adjacent to one another.

10. A method according to any preceding claim, wherein the first balloon layer is made of at least one of: polyamide, polyether block amide, polyethylene, PET, polyurethane.

11. A method according to any preceding claim, wherein the second balloon layer is made of at least one of: polyamide, polyether block amide, polyethylene, PET, polyurethane, all being of a formulation having a lower softening or melting temperature than the material of the first balloon layer.

## Patentansprüche

1. Verfahren zum Ausbilden eines medizinischen Ballons (30, 60, 70, 80), der mit ersten (44, 64, 74, 84) und zweiten Schichten (46, 66, 76, 86) versehen ist, wobei die zweite Schicht die erste Schicht überlagert, wobei die zweite Schicht eine texturierte oder angeraute Außenfläche umfasst; wobei die erste Schicht mit der zweiten Schicht einstückig ist, um die zweite Schicht zu stützen, wobei die zweite Schicht bei Aufblasen des Ballons eine angeraute Außenfläche beibehält; wobei die erste Ballonschicht aus einem Material hergestellt ist, das eine erste Erweichungs- oder Schmelztemperatur und eine erste Nachgiebigkeit aufweist, und die zweite Ballonschicht aus einem Material hergestellt ist, das eine zweite Erweichungs- oder Schmelztemperatur und eine zweite Nachgiebigkeit aufweist,
wobei das Verfahren die folgenden Schritte umfasst:
Bereitstellen eines Rohschlauchs (140) mit ersten (142) und zweiten Schichten (144) aus den ersten bzw. zweiten Materialien;
Anordnen des Rohschlauchs in einer Form (102), wobei die Form eine texturierte oder angeraute Innenfläche umfasst;
Aufblasen und Erwärmen des Rohschlauchs, um zu bewirken, dass er sich aufbläst, wobei das Erwärmen bewirkt, dass das zweite Material erweicht oder schmilzt;
Drücken der zweiten Schicht gegen die Formoberfläche mittels Druck in dem Rohschlauch, wobei das Drücken bewirkt, dass die zweite Schicht eine texturierte oder angeraute Außenfläche annimmt;
wobei das Aufblasen, Erwärmen und Drücken den Ballon ausbildet; und
Abkühlen und Entleeren des Ballons, wobei die zweite Oberfläche des Ballons die angeraute Außenfläche beibehält,
wobei die erste Ballonschicht und die zweite Ballonschicht **dadurch gekennzeichnet sind, dass** die zweite Erweichungs- oder Schmelztemperatur niedriger ist als die erste Erweichungs- oder Schmelztemperatur und dass
die erste Nachgiebigkeit geringer ist als die zweite Nachgiebigkeit.

2. Verfahren nach Anspruch 1, wobei die zweite Ballonschicht eine Oberflächentextur oder Rauheit von geometrisch geformten Oberflächenmerkmalen aufweist.

3. Verfahren nach Anspruch 2, wobei die geometrisch geformten Oberflächenmerkmale umlaufende Ringe oder Rippen, Nuten, Vorsprünge und/oder Vertiefungen, gezahnte Elemente, zufällige Vorsprünge umfassen.

4. Verfahren nach einem der vorhergehenden Ansprüche, wobei die Rauheit oder Texturierung der Außenfläche des Ballons zwischen 0,2 Ra bis 18 Ra bei einem Aufblasdruck von 1 atm bis 25 atm liegt.

5. Verfahren nach einem der vorhergehenden Ansprüche, wobei die ersten und zweiten Ballonschichten koextrudiert werden.

6. Verfahren nach einem der vorhergehenden Ansprüche, wobei die erste Ballonschicht im Wesentlichen glatte Oberflächen aufweist.

7. Verfahren nach einem der vorhergehenden Ansprüche, wobei die zweite Ballonschicht eine im Wesentlichen glatte Innenfläche aufweist.

8. Verfahren nach einem der vorhergehenden Ansprüche, wobei der Ballon aus zwei Ballonschichten ausgebildet ist.

9. Verfahren nach einem der vorhergehenden Ansprüche, wobei die ersten und zweiten Ballonschichten direkt aneinander angrenzen.

10. Verfahren nach einem der vorhergehenden Ansprüche, wobei die erste Ballonschicht aus mindestens einem von Folgendem hergestellt ist: Polyamid, Polyetherblockamid, Polyethylen, PET, Polyurethan.

11. Verfahren nach einem der vorhergehenden Ansprüche, wobei die zweite Ballonschicht aus mindestens einem von Folgendem hergestellt ist: Polyamid, Polyetherblockamid, Polyethylen, PET, Polyurethan, die alle aus einer Formulierung mit einer niedrigeren Erweichungs- oder Schmelztemperatur als das Material der ersten Ballonschicht bestehen.

## Revendications

1. Procédé de formation d'un ballonnet médical (30, 60, 70, 80) pourvu d'une première couche (44, 64, 74, 84) et d'une deuxième couche (46, 66, 76, 86), la deuxième couche recouvrant la première couche, la deuxième couche comportant une surface externe texturée ou rugueuse ; la première couche étant solidaire de la deuxième couche de manière à supporter la deuxième couche, la deuxième couche conservant une surface externe rugueuse lors du gonflage du ballonnet ; la première couche de ballonnet étant constituée d'un matériau ayant une première température de ramollissement ou de fusion et une première souplesse, et la deuxième couche de ballonnet étant constituée d'un matériau ayant une deuxième température de ramollissement ou de fusion et une deuxième souplesse, le procédé comportant les étapes suivantes :
obtention d'un tube brut (140) ayant une première couche (142) et une deuxième couche (144) desdits premier et deuxième matériaux, respectivement ;
positionnement dudit tube brut dans un moule (102), lequel moule comporte une surface interne texturée ou rugueuse ;
gonflage et chauffage du tube brut de manière à le faire gonfler, ledit chauffage faisant ramollir ou fondre ledit deuxième matériau ;
pression de ladite deuxième couche contre la surface de moule au moyen d'une pression à l'intérieur du tube brut, ladite pression faisant acquérir à ladite deuxième couche une surface externe texturée ou rugueuse ;
lesdits gonflage, chauffage et pression formant ledit ballonnet ; et
refroidissement et dégonflage dudit ballonnet, la deuxième surface du ballonnet conservant ladite surface externe rugueuse,
ladite première couche de ballonnet et ladite deuxième couche de ballonnet étant **caractérisées en ce que**
la deuxième température de ramollissement ou de fusion est plus basse que la première température de ramollissement ou de fusion et **en ce que**
la première souplesse est inférieure à la deuxième souplesse.

2. Procédé selon la revendication 1, dans lequel la deuxième couche de ballonnet a une texture ou rugosité de surface d'éléments de surface de forme géométrique.

3. Procédé selon la revendication 2, dans lequel les éléments de surface de forme géométrique incluent des anneaux ou nervures circonférentiels, des rainures, des saillies et/ou des creux, des éléments dentés, des saillies aléatoires.

4. Procédé selon une quelconque revendication précédente, dans lequel la rugosité ou texture de la surface externe du ballonnet se situe entre 0,2 Ra et 18 Ra à une pression de gonflage allant de 1 atm à 25 atm.

5. Procédé selon une quelconque revendication précédente, dans lequel les première et deuxième couches de ballonnet sont coextrudées.

6. Procédé selon une quelconque revendication précédente, dans lequel la première couche de ballonnet a des surfaces sensiblement lisses.

7. Procédé selon une quelconque revendication précédente, dans lequel la deuxième couche de ballonnet a une surface interne sensiblement lisse.

8. Procédé selon une quelconque revendication précédente, dans lequel le ballonnet est constitué de deux couches de ballonnet.

9. Procédé selon une quelconque revendication précédente, dans lequel les première et deuxième couches de ballonnet sont directement adjacentes l'une à l'autre.

10. Procédé selon une quelconque revendication précédente, dans lequel la première couche de ballonnet est constituée d'au moins un des composés suivants : polyamide, polyéther-bloc-amide, polyéthylène, PET, polyuréthane.

11. Procédé selon une quelconque revendication précédente, dans lequel la deuxième couche de ballonnet est constituée d'au moins un des composés suivants : polyamide, polyéther-bloc-amide, polyéthylène, PET, polyuréthane, tous étant d'une formulation ayant une plus basse température de ramollissement ou de fusion que le matériau de la première couche de ballonnet.
